# EUROPEAN PATENT SPECIFICATION

(11) **EP 4 258 993 B1**
(45) Date of publication and mention of the grant of the patent: **23.04.2025**
(21) Application number: 21823911.9
(22) Date of filing: 08.12.2021
(51) Int. Cl.: A61B 5/16, A61B 5/00

(54) **COMPUTER IMPLEMENTED METHOD AND SYSTEM FOR MAPPING SPATIAL ATTENTION**
COMPUTERIMPLEMENTIERTES VERFAHREN UND SYSTEM ZUR ABBILDUNG RÄUMLICHER AUFMERKSAMKEIT
PROCÉDÉ ET SYSTÈME MIS EN UVRE PAR ORDINATEUR POUR CARTOGRAPHIER L'ATTENTION SPATIALE

(30) Priority: 09.12.2020 EP 20212871
(43) Date of publication of application: 18.10.2023
(73) Proprietor: Universiteit Antwerpen, 2000 Antwerpen (BE); Karel de Grote Hogeschool, 2018 Antwerpen (BE)
(72) Inventor: SAEYS, Wim, 2570 Duffel (BE); TRUIJEN, Steven, 2980 Zoersel (BE); DE BOI, Ivan, 2840 Rumst (BE)
(74) Representative: IP HILLS NV
(86) International application number: PCT/EP2021/084817
(87) International publication number: WO 2022/122834

(56) References cited:
- DVORKIN ASSAF Y ET AL: "Mapping the Neglected Space: Gradients of Detection Revealed by Virtual Reality", vol. 26, 1 February 2012 (2012-02-01), XP055793643, Retrieved from the Internet <URL:https://journals.sagepub.com/doi/pdf/10.1177/1545968311410068> [retrieved on 20210419]
- JANG WONCHEOL ET AL: "Human field of regard, field of view, and attention bias", COMPUTER METHODS AND PROGRAMS IN BIOMEDICINE, ELSEVIER, AMSTERDAM, NL, vol. 135, 19 July 2016 (2016-07-19), pages 115 - 123, XP029709591, ISSN: 0169-2607, DOI: 10.1016/J.CMPB.2016.07.026
- SCHOUTEN BEN ET AL: "The Design of a Virtual Reality Game for Stroke-Induced Attention Deficits", 15 October 2017 (2017-10-15), New York, NY, USA, pages 1 - 230, XP055793637, ISBN: 978-1-4503-5111-9, Retrieved from the Internet <URL:https://dl.acm.org/doi/pdf/10.1145/3130859.3131308?casa_token=UbwQu3OxlI4AAAAA:TN-4_uknYW9CSsea-JEj63pJRXgzNg-gCjj61C-hs7z6BhLlNYNjTB549fNuVso04oINlBes9YtMAg> [retrieved on 20210420], DOI: 10.1145/3130859.3131308
- HUYGELIER HANNE ET AL: "An immersive virtual reality game to train spatial attention orientation after stroke: A feasibility study", 18 September 2020 (2020-09-18), US, pages 1 - 21, XP055793716, ISSN: 2327-9095, Retrieved from the Internet <URL:http://dx.doi.org/10.1080/23279095.2020.1821030> [retrieved on 20210420], DOI: 10.1080/23279095.2020.1821030

## Description

### Field

The present invention generally relates to a computer implemented method and a system for mapping spatial attention.

### Background

Spatial attention is the capacity of someone to process stimuli in his surrounding space. Spatial attention is important for perception, for example for visual perception or auditory perception and can even alter perception, allowing someone to selectively process visual or auditory information by directing attention to a location in space through prioritization of an area within the visual or auditory field.

Unilateral spatial neglect (USN) is an attentional deficit, in particular an impaired awareness for stimuli which cannot be attributed to sensory or motor impairments. This neglect may be due to a medical condition and is for example relatively common after a stroke. Patients may for example fail to orientate, to report or to respond to stimuli, especially to stimuli located on the contra-lesional side of space. The impaired awareness for stimuli may negatively influence independence of a patient during activities of daily living: body orientation may become impaired leading to a high risk of falling or colliding with objects while walking, patients may have difficulties finding products in a shop, patients may fail to eat the food on the neglected side of their plate, they may be dressing only one side of their body, or patients may be unaware of traffic lights when crossing the street.

Well-known and widely spread methods used to assess USN comprise pen-and-paper tasks, such as for example the Star Cancellation Test in which a patient is asked to search an array of figures containing 54 small stars, 52 large stars, 13 letters, and 10 words to locate and cross out the small stars. In another task, known as the Line Bisection test, the patient is asked to use a pencil to mark the midpoint of three different lines on the page.

Even if these tests can assess some cognitive functions such as spatial attention, they suffer from several serious shortcomings. First of all, these tasks are only poorly linked to activities of daily living and can therefore only provide poor understanding of problems the patients suffering from USN are faced with in daily life. Moreover, pen-and-paper tasks are two-dimensional and therefore fail to provide a complete rendering of a patient's USN. Finally, USN is essentially being evaluated in the visual modality in present clinical practice, although deficits of spatial attention have been described in many other sensory modalities: next to visuospatial neglect (VSN) patients may also suffer from auditory or tactile neglect.

To partially overcome these shortcomings, other kinds of assessment may be used, such as behavioural inattention tests or naturalistic action tests, in which someone is asked to perform tasks of daily living such as for example eating, telephone dialling, card sorting, dressing, cleaning one's mouth and many more, while the performed actions are being observed and assessed by a professional. However, this kind of assessments may be relatively time-consuming. Moreover, the presence of an assessor or observer may influence the performance of the patient.

Some of the above-mentioned shortcomings had already been recognized for example, in the scientific paper Dvorkin Assaf et al. (XP055793643). The paper evaluates the use of a rectangular array of targets versus a polar array of targets in a 3D virtual environment, in particular in a 3D room shape with a simple background texture. One static visual targets randomly appeared in space. Subjects were instructed to press a response button as fast as they could whenever they detected a target.

Apart from a medical context, spatial attention may be an important issue in some specific environments, such as for example in traffic. Professional drivers, pilots or, for example air traffic controllers can benefit from a large field of spatial attention given the environment in which they generally work, which may be relatively dense in stimuli, both visual and auditory.

### Summary of the Invention

It is therefore an aim of the present invention to solve or at least alleviate one or more of the above-mentioned problems. In particular, the invention aims at providing a solution for mapping visuospatial attention which is able to provide a relatively complete assessment of spatial attention within a reasonable time span.

To this aim, according to a first aspect of the invention, there is provided a computer implemented method for mapping visuospatial attention. The method comprises the steps of providing a three dimensional scenery on a display to a person, generating stimuli at respective locations in said scenery, measuring, when generating a stimulus of said stimuli, a reaction time between the generating of said stimulus and a spotting by the person of said stimulus, representing said reaction times in function of the locations, thereby visually representing spatial attention of the person.

Contrary to existing two-dimensional tests, such as the Star cancellation test, the present computer implemented method provides a three-dimensional scenery on a screen. Even if the screen itself is two-dimensional, the represented scenery is three-dimensional, i.e. provide some effect of depth of sight, for example a view on a forest, a river, a road, a playground, a kitchen or any other three-dimensional scenery. By measuring reaction time to stimuli in said scenery and visually representing the reaction times in function of the respective locations, a map may be obtained representing spatial attention three-dimensionally. Since the reaction time is measured between the generating and the spotting of the stimuli, no additional potential difficulties are added to the assessment, such as naming of objects, pointing to stimuli, or others, which may have an influence on the result. In prior art assessments, a person may for example spot a stimulus without being able to quickly name it because of word finding problems, which may then negatively influence the assessment. Moreover, in prior art assessment programs, assessment of spatial attention is mostly done in a binary manner: either a stimulus is spotted or not. Contrary to this binary prior art methods, the present method can provide relatively balanced information in that the method can provide a mapping of the reaction time between the generating and the spotting of a stimulus by the user.

The method further includes the steps of feeding said measured reaction times into a statistical model modelling the probability that the stimuli at respective locations are spotted after a predetermined time interval, and obtaining from the statistical model estimated reaction times for further locations in said scenery. Estimated reaction times for further locations may include a mean estimated reaction time as well as a standard deviation or a variance of said mean estimated reaction time for said further locations. The feeding of measured reaction times into a statistical model and obtaining estimated reaction times for further locations can decrease the time needed to perform the mapping of spatial attention while keeping an equal or even higher level of accuracy in that fewer stimuli at fewer locations can be generated. Generating stimuli at as many locations as possible may provide a lot of information on the person's spatial attention but may be relatively time consuming. Decrease in length of such a mapping of spatial attention has turned out to be an important factor since attention of a person may decrease during performance of the mapping.

The stimuli are preferably visual stimuli or auditory stimuli. Visual stimuli may for example include birds appearing in a tree of a forest, fish in a river, cars on a street, a ball or a balloon on a playground, objects in a cupboard or many other. Auditory stimuli may include various sounds, such as shouting, honking, crying, beeping and many others on various volume levels coming from varying angles. The method may be performed with visual stimuli only providing a mapping of visuospatial attention, or the method may be performed with auditory stimuli only providing a mapping of auditory attention. The method may also combine visual and auditory stimuli by providing both stimuli simultaneously at a same location or by providing visual and auditory stimuli at different locations, in which cases the method can provide a mapping of spatial attention including both visual and auditory attention.

The measuring of the reaction time can advantageously comprise tracking a position of the person's pupils. Such tracking can be done with known eye-tracking cameras, preferably one camera per eye. The at least one eye tracking camera is configured to follow a direction of sight of a person's pupil. As soon as the direction of the person's pupils is within a predetermined range, for example of 10% or lower, around a location where a stimulus has been generated, during a predetermined range of time, the method can consider the stimulus as having been spotted by the person. As an example, visually fixing a stimulus for one or two seconds may be considered as spotting the stimulus. For auditory stimuli, the predetermined range where to look to may for example be larger than for visual stimuli, for example up to twice as large, since the direction from where a sound is coming is less easy to fixate by eye. Measuring reaction time in this way has the advantage of being relatively easy for the patient without needing active action from the user which may add any potential difficulty, such as with naming, pointing, pushing a button, or any other indication of spotting of the stimulus.

The display may be a head mounted display. The head mounted display can be configured to provide two different representations of the scenery onto the respective eyes of a person to provide a perception of depth within the displayed scenery. The head mounted display can for example include two displays, in particular one display per eye. A head mounted display can be configured to provide an impression of full immersion of the user in the three-dimensional scenery, which is generally referred to as virtual reality (VR). A head mounted display can provide a compact solution which is able to create a relatively realistic three-dimensional scenery. Alternatively, a stereoscopic view on the three-dimensional scenery may be obtained by displaying anaglyph scenery images and by using anaglyph glasses. It is also possible to obtain an impression of immersion through the use of large screens or displays around the user. Other displays known to the person skilled in the art are possible, as long as they can provide a wide field of view providing an impression of immersion of the user in the three-dimensional scenery.

The representing can for example include a heatmap of the reaction times, including both measured reaction times as well as estimated reaction times. A heatmap is data visualization technique configured to represent a magnitude or a measure of a phenomenon by a colour. A given colour, for example red, may then be attributed to reaction times above a predetermined threshold indicating that a stimulus has not been spotted within a predetermined time range, while other colours, for example green, yellow, orange, or other colours, may be attributed to reaction times smaller than said predetermined threshold. Various colour schemes or colour scales may be used. The heatmap can preferably include an associated heatmap configured to visualize a standard deviation or variance associated to the measured reaction times as well as to the estimated reaction times. Such a heatmap, or set of heatmaps, can allow two- and/or three-dimensional displaying of measured reaction times to stimuli in function of respective locations of said stimuli. The heatmap may for example be a three-dimensional cloud of coloured points or may for example be a two-dimensional slice of a three-dimensional map. Other visualization techniques may be used as well.

The method can further comprise the step of providing at least one cue towards the stimulus. The cues may be visual cues, or auditory cues, or any other sensory cues configured to attract a user's attention towards the stimulus. This cue can for example be a sign, for example a visual sign, such as an arrow or any other indicator, stationary or moving, leading the attention of the user towards the stimulus. A cue may for example be a cue growing in size over time or growing in clarity over time such that a stimulus is first generated without cue, and that a cue is then provided while becoming a 'stronger' cue over time attracting more and more attention of the user towards the stimulus. Such a cue may be especially advantageous when a first mapping of spatial attention has already been carried out. In a next step, the same computer-implemented method may be used in a training program, the providing of the cue being used in areas where suboptimal spatial attention has been measured. However, a cue may also be provided in a very first mapping of spatial attention without any training purpose.

The method may further comprise the step of providing distracting elements within the scenery. The distracting elements can be configured to distract attention of a user away from the stimuli which should be spotted. Said distracting elements may for example include elements which may be related to the stimulus while being different, such as for example other animals popping up when the stimulus to be spotted are only birds. Said distracting elements may also be a familiar element in the scenery, for example people passing by in a street when cars need to be spotted. A difficulty level is preferably adjustable and may be associated with the relative complexity of the scenery with or without moving elements, with the distribution of locations for generating stimuli, with the generation of cues and/or distracting elements, and/or with a speed of successive generation of stimuli. Said level of difficulty can for example be predetermined, or may be adjustable during execution of the method, for example depending on performance.

The statistical model is preferably a Gaussian process. A Gaussian process is not to be confused with a Gaussian distribution. A Gaussian process can model a random process of sampling measured reaction times, which is a function with a continuous domain being time. A Gaussian process can also advantageously be used as a prior probability distribution over functions in Bayesian inference in which it can benefit from properties inherited from the normal distribution. Since the Gaussian process can not only provide an estimate but also an uncertainty of that estimate, for example as a standard deviation or a variance, a relatively reliable prediction of reaction times can be provided with relatively few measured data. If a neural network were used to estimate reaction times, a lot more measured reaction times would be necessary to train said network than when using a Gaussian process.

The obtaining estimated reaction times can comprise performing Gaussian process regression. This method of interpolation can generate an unbiased prediction of intermediate values between measured reaction times at respective locations, when data are spatially related, which is the case for mapping spatial attention. The predicted values preferably include a mean and an associated standard deviation or variance. Other methods of interpolation, such as methods based on smoothness, for example of splines, may also be used, as well as for example non-linear interpolation or methods on biased estimators.

The generating stimuli can advantageously comprise generating a next stimulus where the statistical model includes a high uncertainty, in particular where an uncertainty of an estimated reaction time provided by the statistical model is higher than at other locations or where the uncertainty is higher than a predefined threshold. Estimated reaction times for further locations obtained from the statistical model can include a mean estimated reaction time as well as a standard deviation or variance associated to said mean estimated reaction time. The uncertainty can thus be based on said standard deviation or variance associated to said mean estimated reaction time. In order to decrease the time needed to perform the present method, in particular the steps of generating stimuli at respective locations and measuring respective reaction times to said stimuli, the method may advantageously choose a location for generating a next stimulus based on the uncertainty associated to an estimated reaction time by the statistical model, for example by choosing the location where the uncertainty, in particular standard variation or variance, associated to an estimated reaction time is the highest of all further locations, or alternatively, higher than a predefined threshold, for example higher than one standard deviation, or any other predefined threshold. In fact, the choice for a next location for generating a next stimulus is not a random process. If iterated, the statistical model would choose the same location for generating a next stimulus based on the uncertainty associated to the estimated reaction times. This is in clear opposition to methods in which further stimuli are randomly generated, even if they are generated according to a Gaussian distribution around a given point. Reiterating such a method would lead to a different location for a next stimulus at every iteration, whereas in the present method, the generation of stimuli is determined by the statistical model and is thus not random. By judiciously choosing a location for generating a next stimulus in this way, the method can provide a mapping of spatial attention based on a relatively low number of measurements, which can significantly decrease the time of participation of a person, while providing a relatively high accuracy in the mapping of spatial attention.

Generating a next stimulus may be repeated a predefined number of times, for example 20 times or more or less, to avoid bias in the results due to fatigue of the person spotting the stimuli. Additionally, and/or alternatively, generating a next stimulus may be repeated until estimated reaction times provided by the statistical model have converged, i.e. estimated reaction times do not change significantly anymore between consecutive iterations. In other words, generating a next stimulus does not result anymore in a significant change of estimated reaction times provided by the statistical model anymore, or visually speaking, in a significant change in heatmap.

Additionally, and/or alternatively, the generating stimuli can further comprise generating a next stimulus where a reaction time is measured above a predetermined threshold. As previously mentioned, the method of mapping spatial attention can be further applied for training purposes. A successive mapping of spatial attention can then show an evolution of spatial attention over time. It may then be advantageous when the generation rate of stimuli is higher in locations where measured or estimated reaction times to stimuli are relatively high to obtain a training of reaction to stimuli where needed.

The method can further comprise measuring a user's head movement. A head movement may for example be tracked by a camera or by a gyroscope or by any other detector or sensor of movement or acceleration. The tracking of the head movement may provide additional information on the process of spotting a stimulus. A user may for example first turn the head into a given direction before being able to exactly locate and spot the stimulus and to fix the eyes on the stimulus.

According to a second aspect of the invention, there is provided a system for mapping visuospatial attention comprising a head mounted device including a head mounted display configured to provide a three-dimensional scenery and a controller. The controller comprises at least one processor and at least one memory including computer program code, the at least one memory and computer program code configured to, with the at least one processor, cause the controller to perform the above-described computer implemented method for mapping visuospatial attention. Said method comprises the steps of providing a three-dimensional scenery on a display to a person, generating stimuli at respective locations in said scenery, measuring, when generating a stimulus of said stimuli, a reaction time between the generating of said stimulus and a spotting by the person of said stimulus, representing said reaction times in function of the locations, thereby visually representing spatial attention of the person. The controller is preferably integrated into the head mounted device. The system can provide one or more of the above-mentioned advantages.

The present invention will be further elucidated with reference to figures of exemplary embodiments. Corresponding elements are designated with corresponding reference signs.

### Brief Description of the Drawings

Fig. 1 shows a side view of a preferred embodiment of a system for mapping spatial attention according to an aspect of the invention;
Fig. 2a - 2d show an embodiment of steps of a computer-implemented method for mapping spatial attention according to another aspect of the invention;
Fig. 3a - 3b show an embodiment of the step of representing reaction times in function of the locations of the method of Figures 2a - 2d;
Figure 4 represents a flowchart of a preferred embodiment of the method of Figures 2a - 2d; and
Figure 5 shows a computing system suitable for performing various steps of the method of Figures 2a - 2d.

### Detailed Description of Embodiments

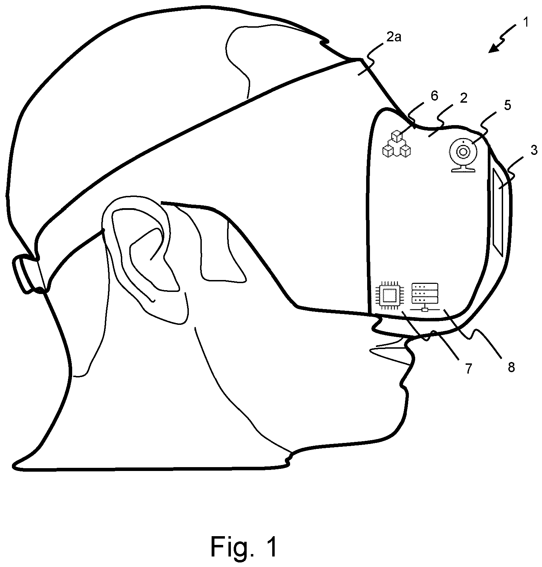

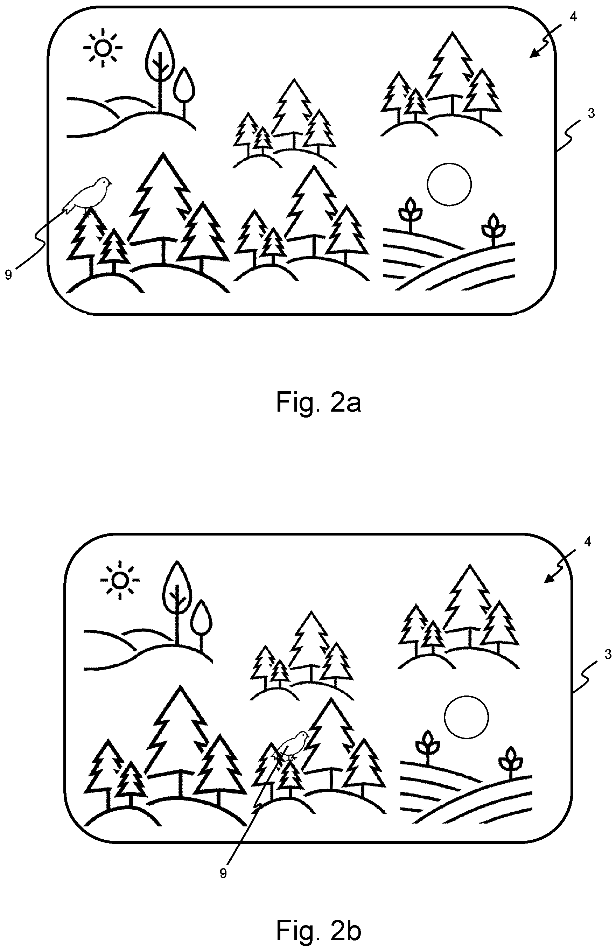

Figure 1 shows a side view of a preferred embodiment of a system 1 for mapping spatial attention according to an aspect of the invention. The system 1 comprises a head mounted device 2 including a head mounted display 3 which is configured to be worn over the eyes and which is fastened to the head, for example via a headband 2a, the display 3 and the headband 2a together forming a single head mounted device 2. The display 3 is configured to provide a three-dimensional scenery 4, as illustrated in Figures 2a - 2d. An impression of three-dimensionality of the scenery 4 can be obtained in various ways, known to the person skilled in the art, for example by displaying stereoscopic images. It is however preferred to provide an impression of full immersion of the user in the three-dimensional scenery, which is generally referred to as virtual reality (VR). The head mounted display 3 can be a single display or a plurality of displays, for example two displays, which can provide two different representations of the scenery 4 onto the respective eyes of a person to provide a perception of depth within the displayed scenery 4. The system 1 further comprises a circuitry 5 arranged to track an eye of a viewer within the displayed scenery 4. Circuitry 5 may for example include an eye tracking camera 5, for example one eye tracking camera per eye. The eye tracking camera 5 may be included into the head mounted device 2 and is configured to follow a direction of sight or point of gaze of a person's pupil, which can for example be done by optical eye tracking using a low-resolution camera which is configured to sense a reflection of light by the eye, in particular by the cornea, for example of infrared or near-infrared light. Eye rotation and a direction of gaze can then be deduced from changes in the reflection of the light. Other eye tracking methods can be used as well, for example using dedicated contact lenses or using an electro-oculogram. The system 1 can further comprise a sensor 6 configured to detect a head movement of the user, for example a gyroscopic sensor or any other known motion detecting sensor. The sensor 6 is preferably also included in the head mounted device 2. Tracking of head movement, eye movement and/or gaze ray can also be used to determine a total area or space in which a person searches for stimuli to be spotted, as will be explained further.

The system 1 further comprises at least one controller comprising at least one processor 7 and at least one memory 8 including computer program code. The at least one memory 8 and computer program code are configured to, with the at least one processor 7, cause the controller to perform the method for mapping spatial attention. The controller is preferably at least partly integrated into the head mounted device 2. The head mounted device 2 may also be wirelessly or wiredly connectable to an external and/or remote controller, for example including additional memory to store results of the method for mapping spatial attention. In case of mapping auditory spatial attention, the system 1 may also include headphones, which are not illustrated here, and which may optionally be integrated into the head mounted device 2.

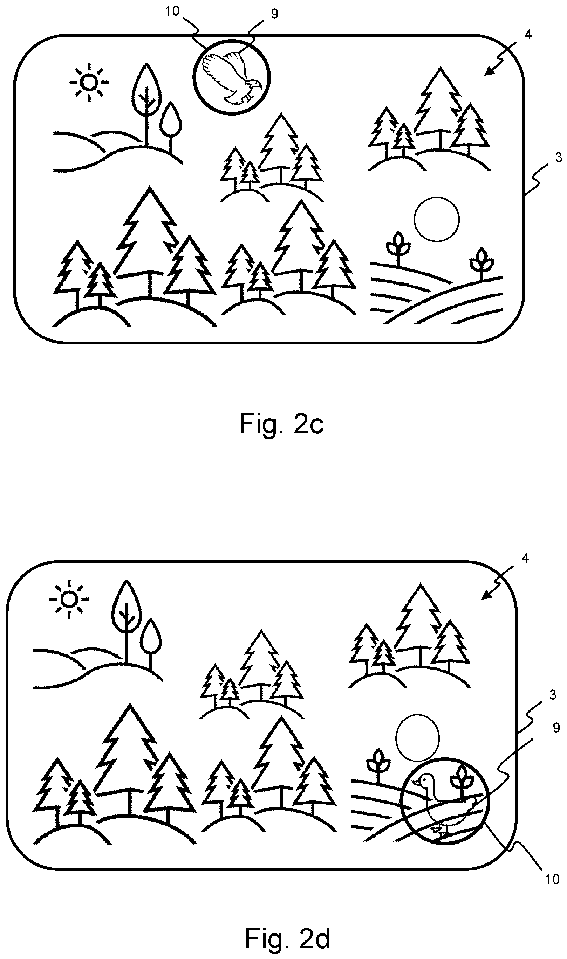

Fig. 2a - 2d show an embodiment of some steps of the computer-implemented method for mapping spatial attention. The method comprises the steps of providing a three-dimensional scenery 4 on a display 3 to a person, for example on a head mounted display 3 as shown in Figure 1. In Figures 2a - 2d the scenery 3 is a hilly landscape, partly a forest, partly fields. The scenery can also be any other kind of scenery, such as streets with buildings along, a road or a river in a countryside, a town centre, a crossroad, a picknick-table in the countryside, a library or any other scenery, as long as the scenery, in combination with the way of displaying, can provide an impression of depth. Said scenery 3 may be stored locally on memory included in the head mounted device 2 or remotely on a computing system, from which the scenery can be transmitted to the head mounted device 2, via a wireless or wired connection. Instead of being displayed on a head mounted display, said scenery 4 may also be displayed on relatively large screens around a person such that the person can also get an impression of being immersed in the scenery. The scenery 4 can be static or moving. The method further comprises the step of generating stimuli 9 at respective locations in said scenery 4. Said respective locations may be logged in any known three-dimensional coordinate system. In the present examples, the stimuli 9 are visual stimuli, but also auditory stimuli are possible. As an example, birds can appear in the scenery 4, or the singing of birds may be generated at respective locations in the scenery 4, which is an example of an auditory stimulus. Many other possible visual or auditory stimuli can be imagined, such for example fish appearing in an underwater scene, people popping up and/or screaming in a town scenery, balloons flying by, food appearing on a picknick table and many more. Stimuli are preferably generated successively rather than simultaneously, so that the user has time to perceive and spot said stimuli 9 one by one. In the present example, a first bird may pop up on a left side of the scenery, as shown in Figure 2a, then another bird may appear around a lower middle of the scenery 4 (Figure 2b). Next, a flying bird may appear in the sky, as shown in Figure 2c, and then another bird may appear towards the right side of the scenery 4, as seen in Figure 2d. Many variations in the appearance of the stimuli 9 are possible. Stimuli may be generated at various depths, depending on the scenery, for example in a range around one meter, or around approximately ten meters, or in between, or closer or further, in a mixed way or in separate test sessions. The order of appearance and the location of appearance may be predetermined, or may be adjustable, for example by a person surveying the mapping of spatial attention. The direction of looking or the point of gaze of the user may for example be followed by the eye tracking camera 5.

The method further comprises the step of measuring, when generating a stimulus of said stimuli 9, a reaction time between the generating of said stimulus and a spotting by the person of said stimulus. The user is said to have spotted a generated stimulus when the user looks at the stimulus 9, as determined for example by the eye tracking camera 5, for a pre-determined amount of time, for example for at least 1 second or 2 seconds or more or less. Said amount of time to define the spotting may be adjustable. The spotting of the stimulus by the person may include a predetermined spatial range or solid angle around the location where the stimulus has been generated to account for a potential error margin of the eye tracking. The eye tracking may take into account a gaze ray, head movement and/or eye movement. If an accuracy of the direction of gaze of the person is expressed as a scalar product of the direction of gaze and the direction of the position or location of the generated stimulus, then said scalar product should be 1 when the person looks exactly at the location of the stimulus and 0 when the person looks at an angle of 90° from the direction of the location where the stimulus has been generated. The accuracy of the spotting may for example be chosen to be above 0.9 to be qualified as a spotting of the stimulus by the person. The accuracy may also be chosen higher, for example to be above 0.95 or above 0.99 to qualify as a spotting of the stimulus. Said accuracy can be adjustable. A user may not spot a generated stimulus at all, for example because it is generated at a location which is relatively far away of the previous stimulus, because of inattention, or because the user may suffer from a form of spatial neglect at that location. The method may thereto include an upper range or time limit, above which time limit the stimulus disappears again and is marked as not spotted, for example after 10 seconds, after 15 seconds, or more or less, which upper time limit may also be adjustable. The method can also include at least one cue 10 towards the stimulus 9. A cue 10 may for example be a growing arrow, or a circle filling up around the stimulus, or any other cue which may attract attention of the user and direct said attention towards the stimulus 9.

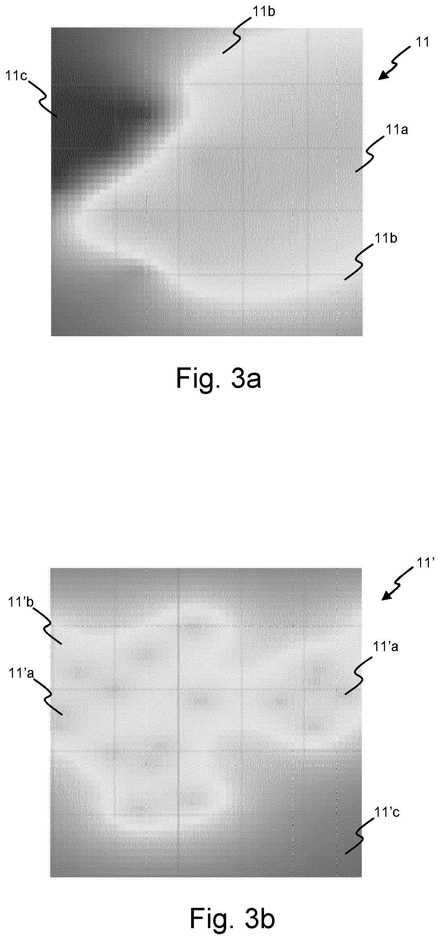

Fig. 3a - 3b show an embodiment of the step of representing reaction times in function of the locations of a generated stimulus 9 to visually represent spatial attention of the person according to the method for mapping spatial attention. This representation of reaction times may for example be done by a heatmap 11, 11' in which a colour code can be indicative for a predetermined range of reaction times, as for example in heatmap 11, or indicative for a predetermined range of standard deviations associated to said reaction times, as for example in heatmap 11'. As an example, a first colour 11a may indicate a relatively swift spotting of the stimulus 9 at the respective location in the scenery 4, for example within 3 seconds of the generation of the stimulus 9. A second colour 11b may indicate a spotting within a range of for example 4 to 10 seconds, whereas a third colour 11c may indicate a reaction time over 10 seconds. Since these colours 11a, 11b and 11c represent a reaction time, both measured reaction times as well as estimated reaction times, in function of the location of the respective stimulus, a map is obtained which visually represents spatial attention of the user: areas coloured in the third colour 11c refer to locations where a stimulus has been spotted relatively late or not at all, indicating areas of what can be called spatial neglect. Other ranges of reaction times may be used, smaller ranges and/or larger ranges, in combination with a plurality of colours or a continuous scale of colours. In heatmap 11', a first colour 11'a may indicate a relative high certainty, or low standard deviation, on the reaction time for the spotting of the stimulus at the respective location in the scenery, for example because the reaction time of the spotting of the stimulus has been measured rather than estimated at the respective location. A second colour 11'b may indicate a reasonable uncertainty or a standard deviation below a predefined threshold on the reaction time, for example because the reaction time at the respective location results from an estimation of the reaction time by the statistical model, for example at a location in between or close to locations with measured reaction times. A third colour 11'c may be indicative of a relatively high uncertainty, or a relatively high standard deviation on the estimated reaction times, for example because no stimulus has been generated at or near the respective locations. In the present figures, the visual representation 11, 11' is a two-dimensional representation, whereas the scenery 4 in which the stimuli 9 are generated are three-dimensional. The two-dimensional representations of Figures 3a and 3b may in fact be slices of a three-dimensional heatmap corresponding to the three-dimensional scenery 4, or they may be projections of part of a sphere on a two-dimensional map when the field of view of the user is represented as a sphere. Three-dimensional heatmaps may also be used directly for the representation of the reaction times in function of the locations of the generated stimuli. As can be seen in the heatmaps 11, 11', the representation does not only include measured reaction times, but also estimated reaction times, as will be explained further.

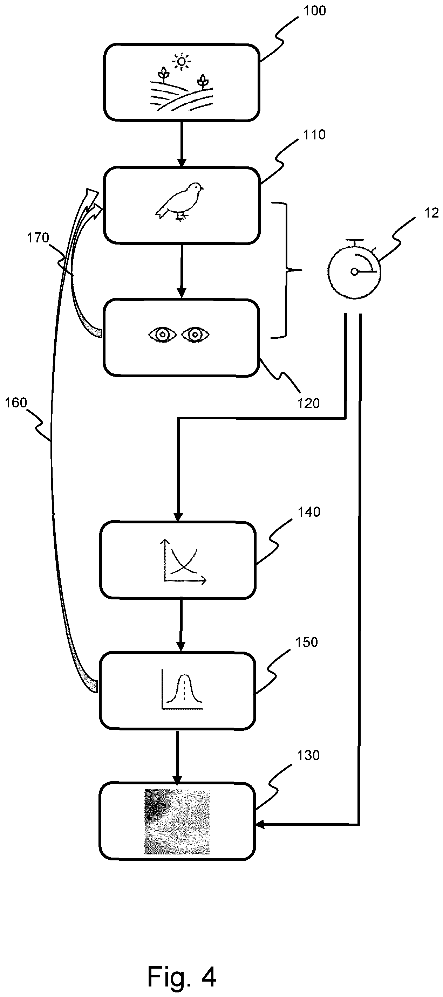

Figure 4 represents a flowchart of a preferred embodiment of the method for mapping spatial attention. In a first step 100, a three-dimensional scenery 4 is provided on a display to a person, as explained with reference to Figures 2a - 2d. Then in step 110, stimuli 9 are generated at respective locations in said scenery 4. Then when generating a stimulus of said stimuli 9, a reaction time 12 between the generating of said stimulus in step 110 and a spotting by the person of said stimulus in step 120, is measured. A next stimulus is generated, preferably after a spotting 120 of said stimulus by a person. Finally, said reaction times 12 are represented in function of the locations, thereby visually representing spatial attention of the person, as indicated by step 130. This representation may be performed on a remote computing system after wired or wireless transmission of the data to said remote computing system. Said transmission may be performed during or after the preceding steps of generating stimuli and measuring reaction times. Said measured reaction times 12 are then fed into a statistical model 140 modelling the probability that the stimuli at respective locations are spotted after a predetermined time interval. Such a statistical model can for example be a Gaussian process. In a next step 150, estimated reaction times are obtained from the statistical model for further locations of the scenery 4, which may for example be done by performing Gaussian process regression. This step may be performed locally on a processor included in the head mounted device 2 or, after wired or wireless transmission of the measured reaction times, on a remote computing system. Said estimated reaction times may then be represented in function of the locations as well in step 130. The method can further include a feedback step 160: a next stimulus may be generated where the statistical model includes a high uncertainty, in particular where the statistical model includes the highest uncertainty. Generating a next stimulus may be stopped either after a predetermined number of generated stimuli, or after convergence of the estimated reaction times. In other words, when generating a next stimulus does not significantly modify the estimated reaction times obtained from the statistical model anymore, the iterations 160 may be stopped. In this way, the method can provide a relatively reliable mapping without testing every single location in the scenery. The method can also include an additional feedback step 170: a next stimulus may be generated where a reaction time is measured above a predetermined threshold. In this way, the method can be used for training spatial attention and the mapping can visualize progress in training.

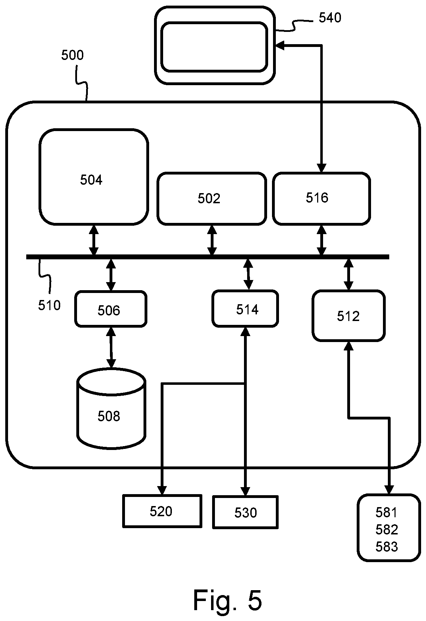

Figure 5 shows a suitable computing system 500 comprising circuitry enabling the performance of steps of embodiments of the method for mapping spatial attention according to an aspect of the invention. The computing system 500 may at least partly be integrated in the head mounted device 2, as previously described. Computing system 500 may in general be formed as a suitable general-purpose computer and comprise a bus 510, a processor 502, a local memory 504, one or more optional input interfaces 514, one or more optional output interfaces 516, a communication interface 512, a storage element interface 506, and one or more storage elements 508. Bus 510 may comprise one or more conductors that permit communication among the components of the computing system 500. Processor 502 may include any type of conventional processor or microprocessor that interprets and executes programming instructions. Local memory 504 may include a random-access memory (RAM) or another type of dynamic storage device that stores information and instructions for execution by processor 502 and/or a read only memory (ROM) or another type of static storage device that stores static information and instructions for use by processor 502. Input interface 514 may comprise one or more conventional mechanisms that permit an operator or user to input information to the computing device 500, such as a keyboard 520, a mouse 530, a pen, voice recognition and/or biometric mechanisms, a camera, etc. Output interface 516 may comprise one or more conventional mechanisms that output information to the operator or user, such as a display 540, etc. Communication interface 512 may comprise any transceiver-like mechanism such as for example one or more Ethernet interfaces that enables computing system 500 to communicate with other devices and/or systems, for example with other computing devices 581, 582, 583. The communication interface 512 of computing system 500 may be connected to such another computing system by means of a local area network (LAN) or a wide area network (WAN) such as for example the internet. Storage element interface 506 may comprise a storage interface such as for example a Serial Advanced Technology Attachment (SATA) interface or a Small Computer System Interface (SCSI) for connecting bus 510 to one or more storage elements 508, such as one or more local disks, for example SATA disk drives, and control the reading and writing of data to and/or from these storage elements 508. Although the storage element(s) 508 above is/are described as a local disk, in general any other suitable computer-readable media such as a removable magnetic disk, optical storage media such as a CD or DVD, -ROM disk, solid state drives, flash memory cards, ... could be used.

As used in this application, the term "circuitry" may refer to one or more or all of the following:
(a) hardware-only circuit implementations such as implementations in only analog and/or digital circuitry and
(b) combinations of hardware circuits and software, such as (as applicable):
   (i) a combination of analog and/or digital hardware circuit(s) with software/firmware and
   (ii) any portions of hardware processor(s) with software (including digital signal processor(s)), software, and memory(ies) that work together to cause an apparatus, such as a mobile phone or server, to perform various functions) and
(c) hardware circuit(s) and/or processor(s), such as microprocessor(s) or a portion of a microprocessor(s), that requires software (e.g. firmware) for operation, but the software may not be present when it is not needed for operation.

This definition of circuitry applies to all uses of this term in this application, including in any claims. As a further example, as used in this application, the term circuitry also covers an implementation of merely a hardware circuit or processor (or multiple processors) or portion of a hardware circuit or processor and its (or their) accompanying software and/or firmware. The term circuitry also covers, for example and if applicable to the particular claim element, a baseband integrated circuit or processor integrated circuit for a mobile device or a similar integrated circuit in a server, a cellular network device, or other computing or network device.

## Claims

1. A computer-implemented method for mapping spatial attention, comprising the steps of:
- providing (100) a three-dimensional scenery (4) on a display (3) to a person;
- generating (110) stimuli (9) at respective locations in said scenery;
- measuring (120), when generating a stimulus of said stimuli, a reaction time (12) between the generating of said stimulus and a spotting by the person of said stimulus;
- representing (130) said reaction times in function of the locations, thereby visually representing spatial attention of the person;
**characterized in that** the method further includes the steps of
- feeding (140) said measured reaction times into a statistical model modelling the probability that the stimuli at respective locations are spotted after a predetermined time interval;
- obtaining (150) from the statistical model estimated reaction times for further locations in said scenery.

2. The method of claim 1, wherein the stimuli are visual stimuli or auditory stimuli.

3. The method according to any of the preceding claims, wherein the measuring of the reaction time comprises tracking a position of the person's pupils.

4. The method according to any of the preceding claims, wherein the display (3) is a head mounted display.

5. The method of any of the preceding claims, wherein the representing includes a heatmap (11, 11') of the reaction times.

6. The method according to any of the preceding claims, further comprising the step of providing at least one cue (10) towards the stimulus.

7. The method of any of the preceding claims, wherein the statistical model is a Gaussian process.

8. The method of any of the preceding claims, wherein the obtaining estimated reaction times comprises performing Gaussian process regression.

9. The method according to any of the preceding claims, wherein the generating stimuli comprises generating a next stimulus (160) where the statistical model includes a high uncertainty.

10. The method according to any of the preceding claims, wherein the generating stimuli further comprises generating a next stimulus (170) where a reaction time is measured above a predetermined threshold.

11. A controller comprising at least one processor (7) and at least one memory (8) including computer program code, the at least one memory and computer program code configured to, with the at least one processor, cause the controller to perform the method according to any of the preceding claims 1-10.

12. A computer program product comprising computer-executable instructions for performing the method according to any of the preceding claims 1-10 when the program is run on a computer.

13. A computer readable storage medium comprising computer-executable instructions for performing the method according to any of the preceding claims 1-10 when the program is run on a computer.

14. System (1) for mapping spatial attention comprising a head mounted device (2) including a head mounted display (3) configured to provide a three-dimensional scenery a circuitry (5) configured to track an eye of a viewer and a controller according to claim 11, wherein the controller is preferably integrated into the head mounted device.

## Patentansprüche

1. Computerumgesetztes Verfahren zum Abbilden der räumlichen Aufmerksamkeit, umfassend folgende Schritte:
- Bereitstellen (100) einer dreidimensionalen Szene (4) an einem Display (3) für eine Person;
- Generieren (110) von Reizen (9) an jeweiligen Stellen in der Szene;
- Messen (120), wenn ein Reiz der besagten Reize generiert wird, einer Reaktionszeit (12) zwischen dem Generieren des Reizes und einer Erkennen des Reizes durch die Person;
- Darstellen (130) der Reaktionszeiten als Funktion der Stellen, wodurch die räumliche Aufmerksamkeit der Person visuell dargestellt wird;
**dadurch gekennzeichnet, dass** das Verfahren ferner folgende Schritte umfasst
- Einspeisen (140) der gemessenen Reaktionszeiten in ein statistisches Modell, das die Wahrscheinlichkeit modelliert, dass die Stimuli an den jeweiligen Stellen nach einem vorbestimmten Zeitintervall erkannt werden;
- Erzielen (150) aus dem statistischen Modell der geschätzten Reaktionszeiten für weitere Stellen in der Szene.

2. Verfahren nach Anspruch 1, wobei die Reize visuelle Reize oder auditorische Reize sind.

3. Verfahren nach einem der vorhergehenden Ansprüche, wobei das Messen der Reaktionszeit das Verfolgen einer Position der Pupillen der Person umfasst.

4. Verfahren nach einem der vorhergehenden Ansprüche, wobei das Display (3) ein am Kopf angebrachtes Display ist.

5. Verfahren nach einem der vorhergehenden Ansprüche, wobei die Darstellung eine Heatmap (11, 11') der Reaktionszeiten umfasst.

6. Verfahren nach einem der vorhergehenden Ansprüche, ferner umfassend den Schritt des Bereitstellens mindestens eines Hinweises (10) auf den Reiz.

7. Verfahren nach einem der vorhergehenden Ansprüche, wobei das statistische Modell ein Gauß-Prozess ist.

8. Verfahren nach einem der vorhergehenden Ansprüche, wobei das Erzielen der geschätzten Reaktionszeiten das Ausführen einer Gauß-Prozess-Regression umfasst.

9. Verfahren nach einem der vorhergehenden Ansprüche, wobei das Generieren der Reize das Generieren eines nächsten Reizes (160) umfasst, wobei das statistische Modell eine hohe Unsicherheit umfasst.

10. Verfahren nach einem der vorhergehenden Ansprüche, wobei das Generieren der Reize ferner das Generieren eines nächsten Reizes (170) umfasst, wobei eine Reaktionszeit oberhalb einer vorbestimmten Schwelle gemessen wird.

11. Steuergerät, umfassend mindestens einen Prozessor (7) und mindestens einen Speicher (8), der Computerprogrammcode umfasst, wobei der mindestens eine Speicher und der Computerprogrammcode dazu konfiguriert sind, das Steuergerät mit dem mindestens einen Prozessor dazu zu veranlassen, das Verfahren nach einem der vorhergehenden Ansprüche 1 bis 10 auszuführen.

12. Computerprogrammprodukt, umfassend computerausführbare Anweisungen zum Ausführen des Verfahrens nach einem der vorhergehenden Ansprüche 1 bis 10, wenn das Programm auf einem Computer läuft.

13. Computerlesbares Speichermedium, umfassend computerausführbare Anweisungen, zum Ausführen des Verfahrens nach einem der vorhergehenden Ansprüche 1 bis 10, wenn das Programm auf einem Computer läuft.

14. System (1) zum Abbilden der räumlichen Aufmerksamkeit, umfassend eine am Kopf angebrachte Vorrichtung (2), die ein am Kopf angebrachtes Display (3) umfasst, das dazu konfiguriert ist, eine dreidimensionale Szene bereitzustellen, Schaltungen (5), die dazu konfiguriert sind, ein Auge eines Betrachters zu verfolgen, und ein Steuergerät nach Anspruch 11, wobei das Steuergerät bevorzugt in die am Kopf angebrachte Vorrichtung integriert ist.

## Revendications

1. Procédé mis en œuvre par ordinateur pour cartographier l'attention spatiale, comprenant les étapes consistant à :
- fournir (100) à une personne une scène tridimensionnelle (4) sur un dispositif d'affichage (3) ;
- générer (110) des stimuli (9) à des emplacements respectifs dans ladite scène ;
- mesurer (120), lors de la génération d'un stimulus desdits stimuli, un temps de réaction (12) entre la génération dudit stimulus et un repérage dudit stimulus par la personne ;
- représenter (130) lesdits temps de réaction en fonction des emplacements, représentant de ce fait visuellement l'attention spatiale de la personne ;
**caractérisé en ce que** le procédé comprend en outre les étapes consistant à
- alimenter (140) lesdits temps de réaction mesurés dans un modèle statistique modélisant la probabilité que les stimuli aux emplacements respectifs soient repérés après un intervalle de temps prédéterminé ;
- obtenir (150) à partir du modèle statistique des temps de réaction estimés pour d'autres emplacements dans ladite scène.

2. Procédé selon la revendication 1, dans lequel les stimuli sont des stimuli visuels ou des stimuli auditifs.

3. Procédé selon l'une quelconque des revendications précédentes, dans lequel la mesure du temps de réaction comprend la poursuite d'une position des pupilles de la personne.

4. Procédé selon l'une quelconque des revendications précédentes, dans lequel le dispositif d'affichage (3) est un dispositif d'affichage monté sur la tête.

5. Procédé selon l'une quelconque des revendications précédentes, dans lequel la représentation comprend une carte thermique (11, 11') des temps de réaction.

6. Procédé selon l'une quelconque des revendications précédentes, comprenant en outre l'étape consistant à fournir au moins un indice (10) vers le stimulus.

7. Procédé selon l'une quelconque des revendications précédentes, dans lequel le modèle statistique est un processus de Gauss.

8. Procédé selon l'une quelconque des revendications précédentes, dans lequel l'obtention des temps de réaction estimés comprend l'exécution d'une régression de processus de Gauss.

9. Procédé selon l'une quelconque des revendications précédentes, dans lequel la génération des stimuli comprend la génération d'un stimulus suivant (160), dans lequel le modèle statistique comprend une incertitude élevée.

10. Procédé selon l'une quelconque des revendications précédentes, dans lequel la génération des stimuli comprend la génération d'un stimulus suivant (170), dans lequel un temps de réaction est mesuré au-dessus d'un seuil prédéterminé.

11. Contrôleur comprenant au moins un processeur (7) et au moins une mémoire (8) comprenant un code programme informatique, ladite au moins une mémoire et le code programme informatique étant configurés pour amener le contrôleur, par ledit au moins un processeur, à effectuer le procédé l'une quelconque des revendications précédentes 1 à 10.

12. Produit de programme informatique comprenant des instructions exécutables par ordinateur pour effectuer le procédé selon l'une quelconque des revendications précédentes 1 à 10 lorsque le programme s'exécute sur un ordinateur.

13. Support de stockage lisible par ordinateur, comprenant des instructions exécutables par ordinateur pour effectuer le procédé selon l'une quelconque des revendications précédentes 1 à 10 lorsque le programme s'exécute sur un ordinateur.

14. Système (1) permettant de cartographier l'attention spatiale, comprenant un dispositif monté sur la tête (2) comprenant un dispositif d'affichage monté sur la tête (3) configuré pour fournir une scène tridimensionnelle, des circuits (5) configurés pour poursuivre un œil d'un observateur, et un contrôleur selon la revendication 11, le contrôleur étant de préférence intégré dans le dispositif monté sur la tête.
